# EUROPÄISCHE PATENTSCHRIFT

(11) **EP 3 174 532 B1**
(45) Veröffentlichungstag und Bekanntmachung des Hinweises auf die Patenterteilung: **13.05.2020**
(21) Anmeldenummer: 15735852.4
(22) Anmeldetag: 03.07.2015
(51) Int. Cl.: A61K 9/20

(54) **PULVERFÖRMIGE, DIREKT VERPRESSBARE POLYVINLALKOHOL-TYPEN**
PULVERULENT, DIRECTLY COMPRESSIBLE TYPES OF POLYVINYL ALCOHOL
TYPES DE POLYALCOOLS DE VINYLE PULVÉRULENTS APTES À LA COMPRESSION DIRECTE

(30) Priorität: 30.07.2014 EP 14002664
(43) Veröffentlichungstag der Anmeldung: 07.06.2017
(73) Patentinhaber: Merck Patent GmbH, 64293 Darmstadt (DE)
(72) Erfinder: OGNIBENE, Roberto, 64297 Darmstadt (DE); BAUER, Finn, 64625 Bensheim (DE); WEDEL, Thorsten, 64589 Stockstadt / Rhein (DE); MODDELMOG, Guenter, 64354 Reinheim (DE)
(86) Internationale Anmeldenummer: PCT/EP2015/001357
(87) Internationale Veröffentlichungsnummer: WO 2016/015814

(56) Entgegenhaltungen:
- WO-A1-88/07366
- WO-A1-97/17947
- US-A1- 2006 177 380

## Beschreibung

Die vorliegende Erfindung betrifft Vormischungen zur Herstellung von wirkstoffhaltigen Tabletten, welche Polyvinylalkohole (PVAs) enthalten. Gegenstand der Erfindung sind auch wirkstoffhaltige Tabletten, die eine entsprechende Vormischung enthalten.

### Stand der Technik

Polyvinylalkohole (PVAs) sind synthetische Polymere, welche in verschiedenen Qualitäten insbes. hinsichtlich Polymerisationsgrad und Viskosität zur Verfügung stehen. Grundsätzlich sind Polyvinylalkohole (PVAs) synthetische, flexible Polymere, welche durch alkalische Hydrolyse von Polyvinylacetat gewonnen werden. Das Polyvinylacetat wiederum erhält man durch eine radikalische Polymerisation aus Vinylacetat. Durch unterschiedliche Kettenlängen und unterschiedliche Hydrolysegrade der Polyvinylacetate kann man Polyvinylalkohole (PVAs) verschiedenster physikalischer Eigenschaften erhalten. Die PVAs werden insbesondere als Filmbildner, Haftgele und als Viskositätsmodulatoren in einer Vielzahl von Anwendungsgebieten z.B. Lacke, Papiere, Textilien, Kosmetika sowie in Pharmazeutica einschl. Drug Delivery Systemen, etc. eingesetzt.

Von besonderem Interesse für die Pharmazeutische Industrie ist die Verwendung der PVAs in pharmazeutischen Zubereitungen wie z.B. in Ophthalmika, als Filmbildner für überzogene Tabletten, als Bindemittel in Granulaten oder als Beschichtungskomponente in Pflastern sowie auch in Drug Delivery Systemen. Ganz besonders interessant ist die Verwendung verschiedener PVA-Typen in der Formulierung von festen oralen pharmazeutischen Darreichungsformen mit einer verlängerten Wirkstofffreisetzung z.B. in sogenannten "Retardtabletten". In diesen Tabletten liegt der Wirkstoff feinverteilt in einer PVA-Matrix vor. Eine verzögerte Wirkstofffreigabe wird in solchen Polymer-haltigen pharmazeutischen Formulierungen dadurch erzielt, dass die Tabletten sich in Gegenwart von Flüssigkeit, wie im Mund oder Magen-Darmtrakt, nicht direkt auflösen sondern quellen und der Wirkstoff durch Diffusion erst nach und nach freigesetzt wird.

Durch solche galenisch modifizierten Tabletten ist es möglich den Wirkstoff aus der Darreichungsform in einer kontrollierten Art und Weise über eine längere Zeit im Körper freizusetzen, um dadurch einen therapeutisch wirksamen Blutspiegel des Medikaments über einen längeren Zeitraum (mehrere Stunden) zu halten. Die beiden wesentlichen Vorteile solcher retardierten Formulierungen sind - im Gegensatz zu Tabletten mit einer sofortigen Wirkstofffreisetzung nach Einnahme - zum einen die Vermeidung von unerwünschten und gegebenenfalls auch toxischen Blut/Plasmaspiegeln des APls als auch eine Reduzierung der Einnahmefrequenz der Tabletten (z.B. statt 3mal/täglich nur noch 1mal/täglich) und somit einer Verbesserung der sog. "Patienten-Compliance" verbunden mit einem verbesserten therapeutischen Ergebnis der medikamentösen Behandlung.

Bekannte Polyvinylalkohole, die für die Verwendung in pharmazeutischen Formulierungen nach den verschiedenen Pharmakopoen (Pharmacopoea Europaea, Ph. Eur.; United States Pharmakopeia (USP), und der Japanischen Pharmakopoe (JP bzw. JPE) spezifiziert sind, sind jedoch nicht oder nur unter besonderen Bedingungen direkt durch Druckeinwirkung tablettierbar. Ein besonderes Problem besteht in diesem Zusammenhang also darin, in einfacher Weise Tabletten herzustellen, die vorwiegend aus entsprechenden PVAs als Wirkstoffträger bestehen, worin der Wirkstoff homogen verteilt ist. Eine direkte Tablettierbarkeit von PVAhaltigen Formulierungen ist üblicherweise in Gegenwart von höheren Anteilen weiterer Bindemittel, wie Laktose, und von Schmiermitteln und ggfs. weiteren Additiven zu erzielen. Häufig werden Formulierungen, in denen PVAs als Wirkstoffträger eingesetzt werden, in Gegenwart von wässrigen oder alkoholischen Lösungen hergestellt. Beispielsweise ist es bekannt, entsprechende Tabletten mit verlängerter Wirkstofffreisetzung herzustellen, indem der Wirkstoff und PVA in Gegenwart von weiteren Zusätzen nach einer Nassgranulation komprimiert werden. Letzteres ist mit dem Nachteil verbunden, dass die erforderlichen Lösungsmittel unter Energieeinsatz wieder entfernt werden müssen.

### Aufgabe

Wie sich aus dem oben Gesagten ergibt, ist es wünschenswert, eine direkt verpessbare Mischung für eine Tablettiermatrix auf der Basis von Polyvinylalkoholen herzustellen, die zur Herstellung von Formulierungen, insbesondere von Tabletten, mit verzögerter Wirkstofffreisetzung einsetzbar ist. Aufgabe der vorliegenden Erfindung ist es somit auch, Polyvinylalkohole enthaltende Tabletten mit verzögerter Wirkstoffabgabe zur Verfügung zu stellen.

Durch die Verwendung einer Kombination von feinkörnigen, gegebenenfalls gemahlenen, Polyvinylalkoholen, die an sich nur eine geringe Kompressibilität aufweisen, mit mikrokristallinen Cellulosen wird nun überraschend eine direkt verpessbare Mischung als Tablettiermatrix erhalten, die zur Herstellung von Formulierungen mit verzögerter Wirkstofffreisetzung verwendet werden kann.

### Kurze Beschreibung der Erfindung

Durch die vorliegende Erfindung wird dem Galeniker eine Vormischung zur Herstellung von wirkstoffhaltigen Tabletten zur Verfügung gestellt, die eine Co-Mischung enthält aus Polyvinylalkoholen (PVAs), welche mittlere Korngrößen <100 µm aufweisen, und mikrokristallinen Cellulosen (MCCs). Vorzugsweise sind solche Vormischungen Gegenstand der vorliegenden Erfindung, welche mikrokristalline Cellulosen mit einer mittleren Korngröße von ≤150 µm, bevorzugt mit einer mittleren Korngröße im Bereich von 100 µm bis 140 µm enthalten. Besonders gute Eigenschaften weisen direkt verpressbare Vormischungen auf, worin Polyvinylalkohole mit einer mittleren Korngröße im Bereich von 80 µm bis 90 µm enthalten sind, so dass die Lösung der Aufgabe der vorliegenden Erfindung insbesondere durch entsprechende direkt verpressbare Co-Mischungen erfolgt. Bei den Vormischungen handelt es sich erfindungsgemäß um Co-Mischungen aus PVA mit mikrokristallinen Cellulosen im Gewichtsverhältnis 2:1 bis 1:2, bevorzugt in einem Verhältnis von 2:1 bis 1:1. Erfindungsgemäß handelt es sich bei den Vormischungen um Co-Mischungen aus mikrokristallinen Cellulosen (MCCs) und zur Wirkstoffretardierung geeigneten Polyvinylalkoholen (PVAs), wobei letztere die Anforderungen der Pharmakopöen (Ph. Eur., USP/NF und JPE erfüllen. Erfindungsgemäße Vormischungen in Form von Co-Mischungen enthalten mikrokristallinen Cellulosen (MCCs) und zur Wirkstoffretardierung geeigneten Polyvinylalkoholen (PVAs) der Typen 18-88, 26-88 und 40-88 gemäß den Anforderungen der Pharmakopöen Ph. Eur., USP/NF und JPE, sowie des Typs 28-99 gemäß den Anforderungen der JPE und Ph. Eur.. Vorzugsweise bestehen diese Co-Mischungen aus mikrokristallinen Cellulosen (MCCs) und zur Wirkstoffretardierung geeigneten Polyvinylalkoholen (PVAs) der Typen PVA 26-88 und PVA 40-88. Entsprechende Vormischungen können durch Kompression bei einer Preßkraft von 20 kN zu Tabletten mit Härten von >380 N geformt werden, die und eine Friabilität ≤0.1 Gew.% aufweisen. Durch Kompression bei einer Preßkraft von 10 kN lassen sie sich zu Tabletten mit Härten von >178 N formen, die eine Friabilität ≤0.1 Gew.% aufweisen.

Gegenstand der vorliegenden Erfindung sind aber auch wirkstoffhaltige Tabletten, welche eine Trägermatrix bestehend aus Polyvinylalkoholen und mikrokristallinen Cellulosen enthalten. Besonders gute Eigenschaften zeigen entsprechende Tabletten mit verlängerter Wirkstofffreisetzung, die eine solche Vormischung aus mikrokristallinen Cellulosen (MCCs) und Polyvinylalkoholen (PVAs) wie oben charakterisiert, enthalten in einer Menge von 1 - 99 Gew.-%, bevorzugt in einer Menge von 5 - 95 Gew.-%, ganz besonders bevorzugt in einer Menge von 10 - 90 Gew.-% bezogen auf das Gesamtgewicht der Tablette. Vorteilhafter Weise können aus den erfindungsgemäßen Vormischungen wirkstoffhaltige Tabletten durch Kompression bei niedrigen Preßkräften erhalten werden, wobei die Tabletten, die hohe Tablettenhärten aufweisen, geringe Ausstoßkräfte erfordern. Entsprechende wirkstoffhaltige Tabletten lassen sich durch Kompression bei einer Preßkraft von 20 kN mit Härten von >380 N und einer Friabilität ≦0.1 Gew.% aus den erfindungsgemäßen Vormischungen erhalten, bzw. bei einer Preßkraft von 10 kN können Tabletten mit Härten von >178 N erhalten werden und einer Friabilität ≦50.1 Gew.%. Gegenstand der vorliegenden Erfindung sind insbesondere wirkstoffhaltige Tabletten mit verzögerter Wirkstofffreisetzung, die einerseits die oben beschriebenen Vormischungen enthalten und Wirkstoffe der BCS Klasse I entweder allein oder in Kombination mit anderen Wirkstoffen.

### Detaillierte Beschreibung der Erfindung

Durch die Verwendung einer Kombination von feinkörnigen, gegebenenfalls gemahlenen, Polyvinylalkoholen, die an sich nur eine geringe Kompressibiliät aufweisen, mit mikrokristallinen Cellulosen wird nun überraschend eine direktverpessbare Mischung als Tablettiermatrix erhalten, die zur Herstellung von Formulierungen mit verzögerter Wirkstofffreisetzung verwendet werden kann. Für den Galeniker ist die Verwendung von feinkörnigen PVAs als Retardierungsmatrices besonders erwünscht, da die Feinkörnigkeit die Herstellung von homogeneren Mischungen mit dem zu retardierenden Wirkstoff ermöglicht. Homogene Mischungen wiederum sind für die Einzeldosierungsgenauigkeit ("Content Uniformity") wichtig, d.h. sie sind wichtig für die Verteilung von immer der gleichen Wirkstoffmenge in jede Einzeltablette. Außerdem führen feinkörnige PVA-Partikel und damit verbundene große Partikeloberflächen bei Verwendung in einer Tablette nach der Befeuchtung der Tabletten im Magen-Darm-Trakt zu einer homogeneren Gelschichtbildung, wodurch eine reproduzierbarere und gegebenenfalls auch verlängerte Diffusion des Wirkstoffs durch dieses Gel erzielt werden kann.

Durch Versuche hat sich gezeigt, dass die Direktverpressungseigenschaften von Materialien insbesondere von deren Partikeleigenschaften abhängig sind. Eine entscheidende Rolle neben der Partikelmorphologie und der Sprödigkeit spielt in diesem Zusammenhang vor allem die Partikelgröße. Hier hat sich gezeigt, dass die Korngröße der Retardierungsmatrices auf PVA-Basis von besonderer Bedeutung ist, und zwar wird die Homogenität der Wirkstoffverteilung in der Matrix, sowie der Retardierungseffekt beeinflusst, insbesondere aber auch die Kompressibilität der Matrix.

Um den Einfluß der Korngrößenverteilung zu untersuchen und besonders vorteilhafte mittlere Korngrößen zu ermitteln wurden die pharmakologisch zertifizierten Polyvinylqualitäten PVA 26-88 und 40-88 unter verschiedenen Bedingungen zu je 3 Fraktionen mit unterschiedlichen Korngrößen/Kornverteilungen kalt vermahlen (Fraktion 1: 85-89µm; Fraktion 2: 116-129 µm und Fraktion 3: 207-245 µm). Diese Mahlfraktionen wurden Kompressibilitätsprüfungen in Kombination mit drei im Handel erhältlichen mikrokristallinen Cellulosetypen unterschiedlicher mittlerer Korngrößen unterzogen (Vivapur® 105: ∼25 µm; Vivapur® 102: ∼100 µm und Vivapur® 200: ∼250 µm).

Durch die Untersuchungen wurde überraschend gefunden, dass alle PVA-Partikelfraktionen in Kombination mit einer feinkörnigen MCC-Qualität (∼25 µm) aufgrund des sehr schlechten Fließverhaltens nicht tablettierbar sind. Bei Verwendung von MCC-Typen mit einer mittleren Korngröße von 100µm bzw. 250 µm können bei ausreichender Fließfähigkeit Tabletten guter Härten und mit geringem Abrieb erhalten werden.

Durch die Versuche mit den Qualitäten PVA 26-88 und PVA 40-88 wurde gefunden, dass umso härtere Tabletten erhalten werden je feiner die PVA-Pulver vermahlen wurden.

Beispielsweise werden in den Versuchen mit Mischungen der beiden feinen Pulverfraktionen PVA 26-88 und PVA 40-88 (85-89 µm) mit dem Vivapur® 102 (∼100 µm) die höchsten Tablettenhärten von deutlich höher als 500 N erhalten bei einer Presskraft von 30 kN. Dagegen werden bei Verwendung der gröberen PVA-Fraktionen, d. h. Pulver mit mittleren Partikelgrößen von 116-129 µm, bzw. von 207-245 µm, deutlich weichere Tabletten erhalten. So werden Tabletten mit einer Härte von <300 N bei einer Presskraft von 30 kN bei Verwendung von für PVA 40-88 mit einer Kornfraktion im Bereich von 207-245 µm erhalten, wenn die mikrokristalline Cellulose Vivapur® 200 mit einer mittleren Korngröße von ∼250 µm verwendet wird. Entsprechende Ergebnisse sind im Folgenden in Kompressibilitätsdiagrammen dargestellt.

Insbesondere hat sich gezeigt, dass tendenziell Kombinationen mit der handelsüblichen mikrokristallinen Cellulose Vivapur® 102 mit einer durchschnittlichen Korngrösse von ∼100 µm eine bessere Kompressibilität aufweisen als Mischungen mit dem Vivapur® 200 mit einer durchschnittlichen Korngrösse von ∼100µm ∼250 µm.

Wie die Versuche zeigen, werden durch die besonders guten Verpressungseigenschaften eines Polyvinylalkohols mit mittleren Korngrößen <90 µm, vorzugsweise im Bereich von 80 bis 90 µm, dem Galeniker Polyvinylalkohole zur Verfügung gestellt, die ohne vorherige Granulierung in einem Direktverpressungprozess zur Herstellung von Tabletten einsetzbar sind und durch die Retardtabletten mit optimalen galenischen Eigenschaften erhalten werden. Insbesondere können mittels dieser vorbehandelten PVAs Tabletten mit hoher Härte und geringer Friabilität erhalten werden. Diese Tabletteneigenschaften sind besonders vorteilhaft in der weiteren Handhabung der Tabletten, wie gegebenenfalls erwünschte Einfärbungen in rotierenden Lackierungsanlagen, insbesondere aber beim Abpacken in Blistern, Abfüllen und Transport, aber auch beim Gebrauch durch den Patienten z.B: beim Ausdrücken der Tabletten aus den Blisterverpackungen.

Die Bedingungen zur Herstellung solcher speziellen PVA-Kornfraktionen und deren besonders guten Kompressibilitäten ergeben sich aus den im Folgenden gegebenen Beispielen.

### Abbildungen:

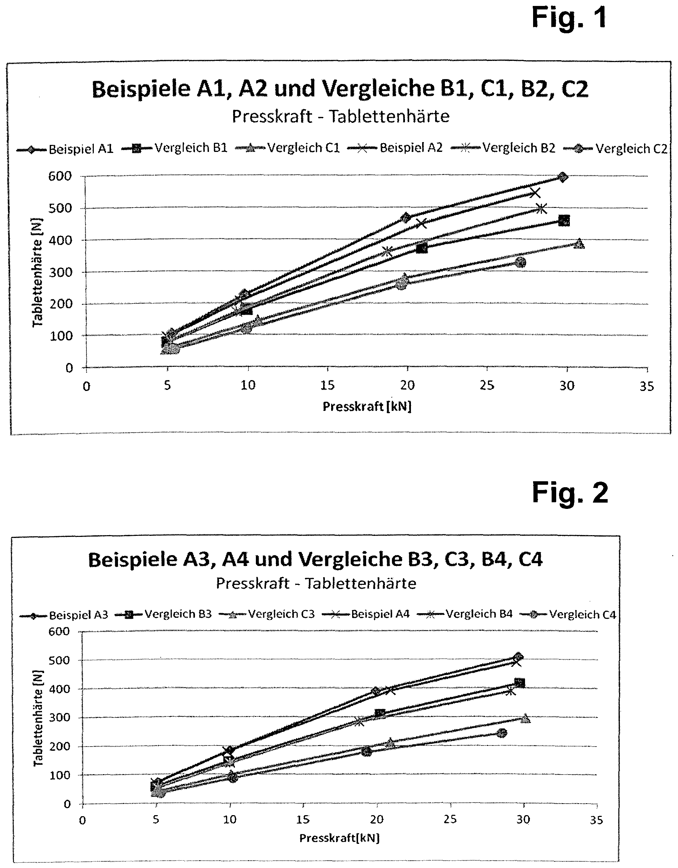

**Fig. 1 und Fig. 2****:** Die Abbildungen Figur 1 und Figur 2 zeigen Charakterisierungen der verschiedenen Polyvinylalkohole PVA 26-88 und 40-88 mit verschiedenen Partikelgrößen vermischt mit MCC und Parteck LUB MST durch Auftragung von Presskraft gegen Tablettenhärte.

### Beispiele

Die vorliegende Beschreibung ermöglicht es dem Fachmann die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, zitierte Veröffentlichungen und gegebenenfalls zitierte Patentliteratur heranzuziehen. Dementsprechend gelten diese Dokumente als Teil der Offenbarung der vorliegenden Beschreibung.

Zum besseren Verständnis und zur Verdeutlichung der Erfindung werden im Folgenden Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew.- bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten daher %-Angaben als Gew.- oder mol-%, mit Ausnahme von Verhältnissen, die in Volumenangaben wiedergegeben sind.

Die in den Beispielen und der Beschreibung sowie in den Ansprüchen gegebenen Temperaturen gelten in °C.

### Geräte/Verfahren zur Charakterisierung der Stoffeigenschaften

1. Schüttdichte: gemäß DIN EN ISO 60: 1999 (Deutsche Fassung) -Angabe in "g/ml"
2. Stampfdichte: gemäß DIN EN ISO 787-11: 1995 (Deutsche Fassung) -Angabe in "g/ml"
3. Schüttwinkel: gemäß DIN ISO 4324: 1983 (Deutsche Fassung) -Angabe in "Grad"
4. Oberfläche bestimmt gemäß BET: Auswertung und Durchführung gemäß Literatur "BET Surface Area by Nitrogen Absorption" von S.Brunauer et al. (Journal of American Chemical Society, 60, 9, 1983) Gerät: ASAP 2420 Fa. Micromeritics Instrument Corporation (USA); Stickstoff; Einwaage: ca. 3,0000 g; Ausheizung: 50°C (5 h); Heizrate 3 K/min; Angabe des arithmetischen Mittelwertes aus drei Bestimmungen
5. Partikelqrößenbestimmung über Laserbeugung mit Trockendispergierung: Mastersizer 2000 mit Dispergiereinheit Scirocco 2000 (Fa. Malvern Instruments Ltd. UK), Bestimmungen bei 1, 2 und 3 bar Gegendruck; Auswertung Fraunhofer; Dispersant RI: 1.000, Obscuration Limits: 0.1-10.0%, Tray Type: General Purpose, Background Time: 7500 msec, Measurement Time: 7500 msec, Durchführung gemäß ISO 13320-1 sowie den Angaben des technischen Handbuchs und den Spezifikationen des Geräteherstellers; Angaben in Vol.-%
6. Partikelgrößenbestimmung über Laserbeugung mit Naßdispergierung: Mastersizer 2000 mit Naßdispergiereinheit Hydro 2000SM (Fa. Malvern Instruments Ltd. UK); Dispersionsmedium Silikonöl dünnflüssig (Hersteller: Evonik Goldschmidt GmbH, Deutschland; Bezeichnung des Herstellers: Tegiloxan3, Artikelnummer des Herstellers: 9000305); Dispersant RI: 1.403; Stirrer Speed: 2500 rpm; Tray Type: General Purpose; Background Time: 7500 msec; Measurement Time: 7500 msec; Obscuration Limits: 7.0-13.0%; Durchführung gemäß ISO 13320-1 sowie den Angaben des technischen Handbuchs und den Spezifikationen des Geräteherstellers; Angaben in Vol%.
   Durchführung: Die Suspensionszelle wird mit dem dünnflüssigen Silikonöl gefüllt, portionsweise die Probe bis zum Erreichen des angestrebten Obscurationbereichs (7,0-13,0%) zugegeben und nach 2 Minuten Wartezeit die Messung gestartet.
   Partikelgrößenbestimmung:
   durch Trockensiebung über einen Siebturm: Retsch AS 200 control, Fa.Retsch (Deutschland); Substanzmenge: ca. 110,00 g; Siebzeit: 30 Minuten; Amplitudenintensität: 1mm; Intervall: 5 Sekunden; Analysensiebe mit Metalldrahtgewebe gemäß DIN ISO 3310; Siebweiten (in pm): 710, 600, 500, 400, 355, 300, 250, 200, 150, 100, 75, 50, 32; Angabe der Mengenverteilung pro Siebfraktion in den Tabellen als "Gew.-% der Einwaage"
7. Die Tablettierungsprüfungen erfolgen folgendermaßen:
   Die Mischungen gemäß der im Versuchsteil angegebenen Zusammensetzungen werden 5 Minuten in einem verschlossenen Edelstahlbehälter (Fassungsvolumen: ca. 2 I, Höhe: ca. 19,5 cm, Durchmesser: ca. 12 cm Außenmaß) auf einem Labor-Taumelmischer (Turbula T2A, Fa.Willy A. Bachofen, Schweiz) gemischt.
   Als Magnesiumstearat wird Parteck LUB MST (Magnesiumstearat pflanzlich) EMPROVE exp Ph. Eur., BP, JP, NF, FCC Artikel Nr. 1.00663 (Merck KGaA, Deutschland) eingesetzt welches über ein 250 µm Sieb abgelegt wurde.
   Die Verpressung zu 500 mg Tabletten (11 mm Stempel, rund, flach, mit Facette) erfolgt auf einer instrumentierten Excenter-Tablettiermaschine Korsch EK 0-DMS (Fa.Korsch, Deutschland) mit dem Auswertesystem Catman 5.0 (Fa.Hottinger Baldwin Messtechnik - HBM, Deutschland).
   Je getesteter Presskraft (Soll-Einstellungen: ∼5, ∼10, ∼20 und ∼30 kN; die effektiv gemessenen Ist-Presskräfte sind in den Beispielen angegeben) werden mindestens 100 Tabletten zur Auswertung der Pressdaten und der galenischen Kennzahlen hergestellt.

Tablettenhärten, Durchmesser und Höhen: Erweka Multicheck 5.1 (Fa. Erweka, Deutschland); Durchschnittsdaten (arithmetische Mittelwerte) aus jeweils 20 Tablettenmessungen pro Presskraft. Die Messungen erfolgen einen Tag nach der Tablettenherstellung.

Tablettenabrieb: Friabilitätsprüfgerät TA420 (Fa. Erweka, Deutschland); Geräteparameter und Ausführung der Messungen gemäß Ph. Eur. 7.Ausgabe "Friabilität von nicht überzogenen Tabletten". Die Messungen erfolgen einen Tag nach Tablettenherstellung.

Tablettenmasse: Multicheck 5.1 (Fa. Erweka, Deutschland) mit der Waage Sartorius CPA 64 (Fa. Sartorius, Deutschland). Angabe des Durchschnittswerts (arithmetischer Mittelwert) aus der Wägung von 20 Tabletten je Presskraft. Die Messungen erfolgen einen Tag nach Tablettenherstellung.

### Charakterisierung der verwendeten Materialien

### 1. Verwendete PVA-Typen und ihre Eigenschaften:

### 1.1 Rohstoffe zur Mahlung

1.1.1. PVA 26-88: Polyvinylalkohol 26-88, geeignet für die Verwendung als Excipient EMPROVE® exp Ph. Eur., USP, JPE, Artikel Nr. 1.41352, Merck KGaA, Darmstadt, Deutschland
1.1.2. PVA 40-88: Polyvinylalkohol 40-88, geeignet für die Verwendung als Excipient EMPROVE® exp Ph. Eur., USP, JPE, Artikel Nr. 1.41353, Merck KGaA, Darmstadt, Deutschland

Diese PVA-Typen liegen als grobkörnige - mehrere Millimeter große - Partikel vor die in dieser Form nicht als eine direkt verpressbare Tablettiermatrix einsetzbar sind.

Die großen Partikel erlauben keine reproduzierbare Befüllung der Stempelmatrizen und somit auch kein konstantes Tablettengewicht bei hohen Rotationsgeschwindigkeiten der (Rundlauf)-Tablettiermaschinen. Außerdem können nur feinkörnige PVAs eine homogene Verteilung des Wirkstoffes -ohne das Auftreten von Entmischungseffekten- in der Tablette gewährleisten; dies ist für die Sicherstellung der Einzeldosiergenauigkeit des Wirkstoffes (content uniformity) in jeder produzierten Tablette unbedingt notwendig. Zusätzlich kann nur durch ein feinkörniges PVA auch die für die reproduzierbare Retardierung notwendige homogene Gelbildung im gesamten Tablettenkörper gewährleistet werden.

Aus diesen Gründen müssen die o.g. grobkörnigen PVA-Typen vor ihrer Verwendung als direkt verpressbare Retardierungsmatrices zerkleinert d.h. gemahlen werden.

Um die hinsichtlich ihrer Kompressibilität optimale Partikelgröße bzw. Partikelgrößenverteilung der beiden PVA-Typen zu ermitteln wurden jeweils 3 Kornfraktionen unterschiedlicher Korngröße durch Kaltvermahlung erzeugt.

### 1.2 Gemahlene PVA-Typen

1.2.1. Gemahlenes PVA 26-88, aus Polyvinylalkohol 26-88 Artikel Nr. 1.41352
mit den mittleren Kornfaktionen Dv50 (Laserbeugung; Trockendispergierung)

| | |
|---|---|
| Fraktion 1: Dv50 | 84,88 - 87,60 µm |
| Fraktion 2: Dv50 | 120,28 -123,16 µm |
| Fraktion 3: Dv50 | 206,86 - 224,48 µm |

1.2.2. Gemahlenes PVA 40-88, aus Polyvinylalkohol 40-88 Artikel Nr. 1.41353
mit den mittleren Kornfaktionen Dv50 (Laserbeugung; Trockendispergierung)

| | |
|---|---|
| Fraktion 1: Dv50 | 85,84 - 87,37 µm |
| Fraktion 2: Dv50 | 115,97 -120,52 µm |
| Fraktion 3: Dv50 | 206,83 - 211,55 µm |

### Vermahlung:

Die Vermahlung der PVA-Typen erfolgt auf einer Aeroplex Spiralstrahlmühle Typ 200 AS der Firma Hosokawa Alpine, Augsburg, Deutschland unter flüssigem Stickstoff als Kaltvermahlung von 0°C bis minus 30°C. Die unterschiedlichen Partikelfraktionen werden insbesondere durch Variation der Mahltemperatur empirisch erzeugt d.h durch laufende in-process-Kontrollen der Partikelgröße werden die Mahlbedingungen so variiert bis die gewünschte Körnung erhalten wird.

Die resultierenden Produkteigenschaften der gemahlenen PVA-Typen insbes. die Pulverkennzahlen wie Schüttdichte, Stampfdichte, Schüttwinkel, BET-Oberfäche, BET-Porenvolumen sowie die Partikelgrößenverteilungen ergeben sich aus den nachfolgenden Tabellen:

### Schüttdichte, Stampfdichte, Schüttwinkel, BET-Oberfläche, BET-Porenvolumen:

### (Details zu den Messverfahren siehe unter Methoden)

| **Muster** | **Schüttdichte (g/ml)** | **Stampfdichte (g/ml)** | **Schüttwinkel (°)** |
|---|---|---|---|
| **PVA 26-88*** 1.Fraktion | 0,51 | 0,70 | 36,7 |
| **PVA 26-88*** 2.Fraktion | 0,54 | 0,72 | 34,2 |
| **PVA 26-88*** 3.Fraktion | 0,57 | 0,74 | 35,2 |
| **PVA 40-88*** 1.Fraktion | 0,51 | 0,70 | 34,0 |
| **PVA 40-88*** 2.Fraktion | 0,53 | 0,71 | 35,0 |
| **PVA 40-88*** 3.Fraktion | 0,56 | 0,72 | 33,8 |

| | | | |
|---|---|---|---|
| * vermahlenes PVA | | | |

| **Muster** | **BET-Oberfläche (m²/g)** | **BET-Porenvolumen (cm³/g)** |
|---|---|---|
| **PVA 26-88*** 1.Fraktion | 0,35 | 0,0019 |
| **PVA 26-88*** 2.Fraktion | 0,26 | 0,0015 |
| **PVA 26-88*** 3.Fraktion | 0,20 | 0,0011 |
| **PVA 40-88*** 1.Fraktion | 0,33 | 0,0018 |
| **PVA 40-88*** 2.Fraktion | 0,22 | 0,0016 |
| **PVA 40-88*** 3.Fraktion | 0,19 | 0,0011 |

| | | |
|---|---|---|
| * vermahlenes PVA | | |

### Partikelverteilung bestimmt über Laserbeugung mit Trockendispergierung (1 bar Gegendruck):

### Angaben in µm (Details zum Messverfahren siehe unter Methoden)

| **Muster** | **Dv5** | **Dv10** | **Dv20** | **Dv25** | **Dv30** | **Dv50** | **Dv75** | **Dv90** | **Dv95** |
|---|---|---|---|---|---|---|---|---|---|
| **PVA 26-88*** 1.Fraktion | 17,39 | 24,78 | 38,52 | 45,59 | 52,97 | 87,60 | 161,70 | 285,80 | 526,72 |
| **PVA 26-88*** 2.Fraktion | 22,81 | 33,47 | 53,87 | 64,26 | 74,92 | 123,16 | 213,12 | 320,40 | 394,31 |
| **PVA 26-88*** 3.Fraktion | 34,27 | 51,46 | 85,84 | 104,56 | 124,33 | 210,80 | 350,73 | 499,99 | 593,56 |
| **PVA 40-88*** 1.Fraktion | 16,33 | 23,54 | 37,10 | 44,13 | 51,49 | 85,96 | 156,09 | 245,33 | 304,05 |
| **PVA 40-88*** 2.Fraktion | 21,56 | 31,96 | 51,45 | 61,20 | 71,15 | 115,97 | 200,37 | 299,76 | 364,57 |
| **PVA 40-88*** 2.Fraktion | 37,50 | 56,52 | 92,13 | 110,24 | 128,68 | 206,83 | 334,62 | 472,78 | 559,87 |

| | | | | | | | | | |
|---|---|---|---|---|---|---|---|---|---|
| * vermahlenes PVA | | | | | | | | | |

### Partikelverteilung bestimmt über Laserbeugung mit Trockendispergierung (2 bar Gegendruck):

### Angaben in µm (Details zum Messverfahren siehe unter Methoden)

| **Muster** | **Dv5** | **Dv10** | **Dv20** | **Dv25** | **Dv30** | **Dv50** | **Dv75** | **Dv90** | **Dv95** |
|---|---|---|---|---|---|---|---|---|---|
| **PVA 26-88*** 1.Fraktion | 16,15 | 23,53 | 37,22 | 44,26 | 51,56 | 85,05 | 151,30 | 240,02 | 305,79 |
| **PVA 26-88*** 2.Fraktion | 21,04 | 31,58 | 52,06 | 62,54 | 73,32 | 122,08 | 213,33 | 320,49 | 390,77 |
| **PVA 26-88*** 3.Fraktion | 31,97 | 48,56 | 81,95 | 100,26 | 119,78 | 206,86 | 350,52 | 508,72 | 613,02 |
| **PVA 40-88*** 1.Fraktion | 15,46 | 22,54 | 36,12 | 43,27 | 50,77 | 85,84 | 156,51 | 247,86 | 309,84 |
| **PVA 40-88*** 2.Fraktion | 20,84 | 31,22 | 51,29 | 61,57 | 72,13 | 120,52 | 215,62 | 344,29 | 457,95 |
| **PVA 40-88*** 3.Fraktion | 36,99 | 55,90 | 92,07 | 110,69 | 129,66 | 209,09 | 336,49 | 472,11 | 556,60 |

| | | | | | | | | | |
|---|---|---|---|---|---|---|---|---|---|
| * vermahlenes PVA | | | | | | | | | |

### Partikelverteilung bestimmt über Laserbeugung mit Trockendispergierung (3 bar Gegendruck):

### Angaben in µm (Details zum Messverfahren siehe unter Methoden)

| **Muster** | **Dv5** | **Dv10** | **Dv20** | **Dv25** | **Dv30** | **Dv50** | **Dv75** | **Dv90** | **Dv95** |
|---|---|---|---|---|---|---|---|---|---|
| **PVA 26-88*** 1.Fraktion | 15,99 | 23,44 | 37,29 | 44,35 | 51,65 | 84,88 | 150,53 | 237,38 | 299,34 |
| **PVA 26-88*** 2. Fraktion | 20,77 | 31,28 | 51,54 | 61,82 | 72,37 | 120,28 | 210,97 | 317,50 | 386,93 |
| **PVA 26-88*** 3.Fraktion | 33,68 | 52,28 | 90,41 | 111,23 | 132,97 | 224,48 | 367,96 | 518,55 | 611,79 |
| **PVA 40-88*** 1.Fraktion | 15,50 | 22,86 | 36,99 | 44,35 | 52,00 | 87,37 | 158,92 | 250,34 | 310,78 |
| **PVA 40-88*** 2. Fraktion | 20,62 | 31,15 | 51,23 | 61,23 | 71,61 | 117,75 | 203,67 | 303,91 | 368,85 |
| **PVA 40-88*** 3. Fraktion | 37,26 | 56,18 | 92,22 | 110,98 | 130,24 | 211,55 | 340,76 | 475,48 | 558,34 |

| | | | | | | | | | |
|---|---|---|---|---|---|---|---|---|---|
| * vermahlenes PVA | | | | | | | | | |

### Partikelverteilung bestimmt über Turmsiebung:

### Angaben in Gewichtsprozent (Details zum Messverfahren siehe unter Methoden)

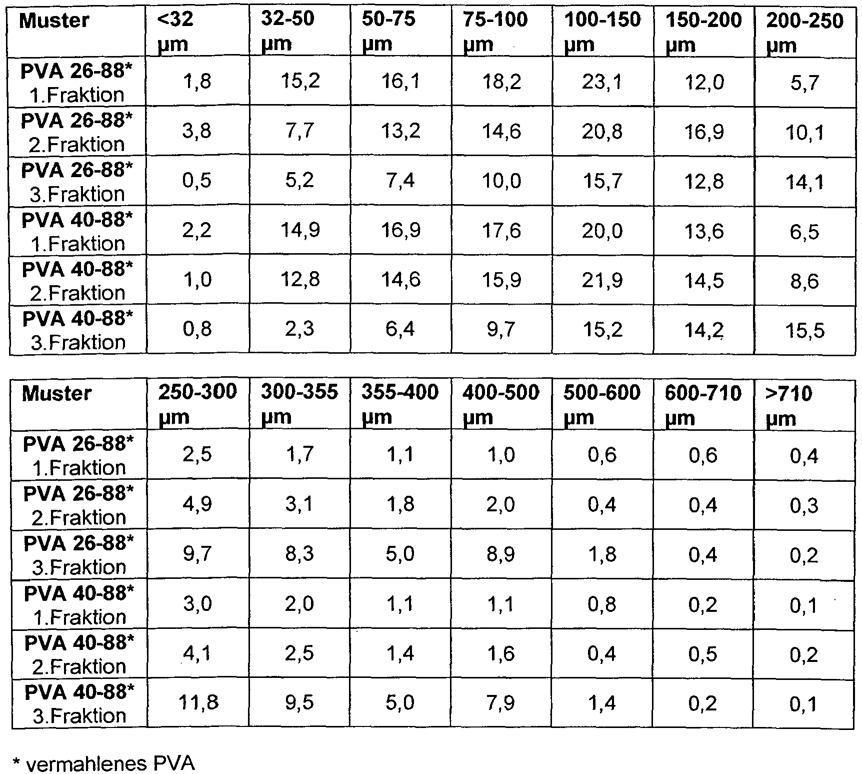

### 2. Mikrokristalline Cellulosen (MCCs)

2.1 Vivapur® Type 200, mikrokristalline Cellulose, Ph. Eur., NF, JP, JRS Pharma, Rosenberg, Deutschland
2.2 Vivapur® Type 102 Premium, mikrokristalline Cellulose, Ph. Eur., NF, JP, JRS Pharma, Rosenberg, Deutschland

### Partikelverteilung bestimmt über Laserbeugung mit Trockendispergierung (1 bar Gegendruck):

### Angaben in µm (Details zum Messverfahren siehe unter Methoden)

| **Muster** | **Dv10** | **Dv20** | **Dv25** | **Dv30** | **Dv50** | **Dv75** | **Dv90** |
|---|---|---|---|---|---|---|---|
| **Vivapur® 102** | 31,56 | 53,04 | 66,00 | 79,89 | 135,87 | 215,53 | 293,94 |
| **Vivapur® 200** | 49,25 | 97,09 | 125,64 | 152,47 | 245,21 | 375,17 | 507,15 |

### Partikelverteilung bestimmt über Laserbeugung mit Trockendispergierung (2 bar Gegendruck):

### Angaben in µm (Details zum Messverfahren siehe unter Methoden)

| **Muster** | **Dv10** | **Dv20** | **Dv25** | **Dv30** | **Dv50** | **Dv75** | **Dv90** |
|---|---|---|---|---|---|---|---|
| **Vivapur® 102** | 27,55 | 45,97 | 57,41 | 70,40 | 127,29 | 208,92 | 288,93 |
| **Vivapur® 200** | 44,08 | 86,21 | 113,63 | 140,90 | 235,62 | 365,86 | 497,34 |

### Partikelverteilung bestimmt über Laserbeugung mit Trockendispergierung (3 bar Gegendruck):

### Angaben in µm (Details zum Messverfahren siehe unter Methoden)

| **Muster** | **Dv10** | **Dv20** | **Dv25** | **Dv30** | **Dv50** | **Dv75** | **Dv90** |
|---|---|---|---|---|---|---|---|
| **Vivapur® 102** | 23,61 | 38,84 | 48,19 | 59,22 | 114,76 | 198,37 | 278,99 |
| **Vivapur® 200** | 138,43 | 73,36 | 97,85 | 124,94 | 223,50 | 356,46 | 490,73 |

### Partikelverteilung bestimmt über Laserbeugung mit Nassdispergierung (in dünnflüssigem Silikonöl):

### Angaben in µm (Details zum Messverfahren siehe unter Methoden)

| **Muster** | **Dv10** | **Dv20** | **Dv25** | **Dv30** | **Dv50** | **Dv75** | **Dv90** |
|---|---|---|---|---|---|---|---|
| **Vivapur® 102** | 28,28 | 47,27 | 58,07 | 69,46 | 119,03 | 200,35 | 285,42 |
| **Vivapur® 200** | 33,53 | 59,12 | 74,18 | 90,77 | 171,42 | 302,56 | 434,89 |

### 3. Übrige Materialien

3.1 Parteck LUB MST (Magnesiumstearat pflanzlich) EMPROVE exp Ph. Eur., BP, JP, NF,
   FCC Artikel Nr. 1.00663 (Merck KGaA, Deutschland)
3.2 Siliciumdioxid kolloidal, hochdispers geeignet für die Verwendung als Excipient
   EMPROVE exp Ph. Eur., NF, JP, E 551 Artikel Nr. 1.13126 (Merck KGaA,
   Deutschland)

### Versuchsergebnisse

Durch die folgenden Versuche wurde gefunden, dass die Körnung des verwendeten PVAs einen erheblichen Einfluss auf das Pressverhalten (Presskraft-Tablettenhärte-Verhältnis) hat.

### A) Ergebnis:

Es wurde gefunden, dass Co-Mischungen auf Basis von gemahlenen Polyvinylalkoholen mit einer Dv50 von 70-90 µm gegenüber gröberen PVA-Kornfraktionen in Kombination mit mikrokristallinen Cellulosen (MCCs) eine besonders gute Kompressibilität aufweisen. So lassen sich in dem Beispiel A1 Tablettenhärten von >400 N bei 20 kN Presskraft und sogar Härten von >500 N bei 30 kN Presskraft erhalten. Auch in dem Beispiel A3 werden Tablettenhärten von >350 N (bei 20 kN Presskraft) bzw. >450 N (bei 30 kN Presskraft) erzielt..

Diese speziellen PVA-MCC-Co-Mischungen sind damit als Matrices zur Formulierung von Retardtabletten in Kombination mit an sich schlecht verpressbaren Wirkstoffen in der Direkttablettierung besonders gut geeignet.

### B) Vorgehensweise:

1. Herstellung der Abmischungen bestehend aus den beiden mikrokristallinen Cellulosen des Marktes mit den jeweiligen PVA-Kornfaktionen im Mengenverhältnis 1:1.
2. Nach 5 Minuten Mischen im Turbulamischer werden 0,25 Gew.% hochdisperses Siliciumdoxid zugegeben und nochmals 5 Minuten gemischt. Danach wird die Mischung über ein 800 µm Handsieb abgelegt.
3. Nach Zusatz von 0,25 Gew.% Parteck® LUB MST wird nochmals 5 Minuten gemischt und anschließend verpresst.
4. Die Tablettencharakterisierung erfolgt hinsichtlich der Parameter Tablettenhärte, Tablettenmasse, Tablettenhöhe, Tablettenabrieb sowie notwendiger Ausstoßkraft

### C) Versuchsergebnisse:

### 1a. Herstellung der Abmischungen der mikrokristallinen Cellulose Vivapur® 102 Premium mit den 3 Kornfraktionen PVA 26-88 und PVA 40-88

Allgemeine Beschreibung: die jeweiligen Kornfraktionen PVA 26-88 und PVA 40-88 werden zur Entfernung von eventuellen Grobanteilen und Agglomeraten durch ein 800 µm Handsieb abgelegt. 300 g dieser gesiebten Ware werden in ein 21 Turbulamischgefäß eingewogen, 300 g der mikrokristallinen Cellulose Vivapur® 102 Premium hinzugegeben und 5 min. in einem Turbulamischer T2A gemischt.

**Tabelle 1a: Zusammensetzung der Co-Mischungen der gemahlenen PVA-Fraktionen mit der mikrokristallinen Cellulose Vivapur 102 Premium**

| **Zusammensetzung** | **50 Gew.% PVA** | **50 Gew.% MCC** |
|---|---|---|
| Beispiel A1 | PVA 26-88* 1.Fraktion | Vivapur® 102 Premium |
| Vergleich B1 | PVA 26-88* 2.Fraktion | Vivapur® 102 Premium |
| Vergleich C1 | PVA 26-88* 3.Fraktion | Vivapur® 102 Premium |
| Beispiel A2 | PVA 40-88* 1.Fraktion | Vivapur® 102 Premium |
| Vergleich B2 | PVA 40-88* 2.Fraktion | Vivapur® 102 Premium |
| Vergleich C2 | PVA 40-88* 3.Fraktion | Vivapur® 102 Premium |

| | | |
|---|---|---|
| *: vermahlenes PVA | | |

### 1b. Abmischungen mit hochdispersem Siliciumdioxid

Zur Verbesserung des Rieselfähigeit werden den Beispielen und Vergleichen jeweils 0,25 Gew.% hochdisperses Siliciumdioxid zugegeben und nochmals 5 Minuten vermischt.

### 1c. Verpressung dieser Abmischungen und Tablettencharakterisierung

Allg. Beschreibung: je 498,75 g der oben hergestellten Co-Mischungen aus den Beispielen A1 und A2 bzw. den Vergleichen B1, C1, B2 und C2 werden in einem Turbulamischgefäß mit 1,25 g Magnesiumstearat versetzt, nochmals 5 min. in einem Turbulamischer T2A gemischt und an einer Exzenterpresse Korsch EK 0-DMS tablettiert.

**Tabelle 1b: Tablettierdaten der Co-Mischungen der gemahlenen PVA-Fraktionen mit der mikrokristallinen Cellulose Vivapur® 102 Premium**

| Legende: | | | | | | | |
|---|---|---|---|---|---|---|---|
| A: Presskraft [kN] | | | | B: Tablettenhärte nach 1 Tag [N] | | | |
| C: Tablettenmasse [mg] | | | | D: Tablettenhöhe [mm] | | | |
| E: Abrieb [%] | | | | F: Ausstoßkraft(N) | | | |
| | | | | | | | |

| **Muster** | **A** | | **B** | **C** | **D** | **E** | **F** |
|---|---|---|---|---|---|---|---|
| | **Soll** | **Ist** | | | | | |
| **Beispiel A1** | 5 | 5,3 | 104,8 | 501,4 | 5,6 | 0,05 | 113,8 |
| | 10 | 9,8 | 226,8 | 503,8 | 4,9 | 0 | 114,0 |
| | 20 | 19,9 | 465,2 | 506,1 | 4,5 | 0 | 66,1 |
| | 30 | 29,7 | 593,5 | 505,2 | 4,4 | 0 | 50,0 |
| **Vergleich B1** | 5 | 5,0 | 75,6 | 496,1 | 5,6 | 0,13 | 104,4 |
| | 10 | 9,9 | 177,9 | 495,3 | 4,9 | 0 | 109,3 |
| | 20 | 20,9 | 372,6 | 496,8 | 4,5 | 0 | 67,3 |
| | 30 | 29,8 | 459,6 | 497,6 | 4,4 | 0 | 55,4 |
| **Vergleich C1** | 5 | 4,9 | 55,6 | 499,6 | 5,5 | 0,25 | 108,7 |
| | 10 | 10,6 | 144,6 | 500,8 | 4,8 | 0 | 116,2 |
| | 20 | 19,8 | 278,0 | 500,2 | 4,5 | 0 | 84,3 |
| | 30 | 30,7 | 388,5 | 498,2 | 4,3 | 0 | 68,0 |
| **Beispiel A2** | 5 | 5,0 | 92,4 | 497,7 | 5,5 | 0,05 | 111,9 |
| | 10 | 9,5 | 207,2 | 496,7 | 4,8 | 0 | 118,6 |
| | 20 | 20,9 | 447,7 | 498,7 | 4,3 | 0 | 68,4 |
| | 30 | 28,0 | 544,4 | 500,3 | 4,3 | 0 | 55,3 |
| **Vergleich B2** | 5 | 5,2 | 80,3 | 497,8 | 5,7 | 0,04 | 117,9 |
| | 10 | 9,3 | 171,7 | 499,6 | 5,1 | 0 | 126,4 |
| | 20 | 18,7 | 360,7 | 502,1 | 4,6 | 0 | 85,6 |
| | 30 | 28,4 | 495,4 | 507,0 | 4,6 | 0 | 63,9 |
| **Vergleich C2** | 5 | 5,4 | 53,7 | 502,5 | 5,7 | 0,28 | 104,1 |
| | 10 | 9,9 | 119,2 | 502,6 | 5,1 | 0 | 114,4 |
| | 20 | 19,6 | 255,4 | 497,3 | 4,6 | 0 | 84,4 |
| | 30 | 27,1 | 326,8 | 496,7 | 4,5 | 0 | 71,5 |

In der Figur 1 sind die stark unterschiedlichen Presskraft-Tablettenhärte-Profile zur besseren Veranschaulichung grafisch dargestellt.

### 2a. Herstellung der Abmischungen der mikrokristallinen Cellulose Vivapur® 200 mit den 3 Kornfraktionen PVA 26-88 und PVA 40-88

Allgemeine Beschreibung: die jeweiligen Kornfraktionen PVA 26-88 und PVA 40-88 werden zur Entfernung von eventuellen Grobanteilen und Agglomeraten durch ein 800 µm Handsieb abgelegt. 300 g dieser gesiebten Ware werden in ein 21 Turbulamischgefäß eingewogen, 300 g der mikrokristallinen Cellulose Vivapur® 200 hinzugegeben und 5 min. in einem Turbulamischer T2A gemischt.

**Tabelle 2a: Zusammensetzung der Co-Mischungen der gemahlenen PVA-Fraktionen mit der mikrokristallinen Cellulose Vivapur® 200**

| **Zusammensetzung** | **50 Gew.% PVA** | **50 Gew.% MCC** |
|---|---|---|
| Beispiel A3 | PVA 26-88* 1.Fraktion | Vivapur® 200 |
| Verpleich B3 | PVA 26-88* 2.Fraktion | Vivapur® 200 |
| Vergleich C3 | PVA 26-88* 3.Fraktion | Vivapur® 200 |
| Beispiel A4 | PVA 40-88* 1.Fraktion | Vivapur® 200 |
| Vergleich B4 | PVA 40-88* 2.Fraktion | Vivapur® 200 |
| Vergleich C4 | PVA 40-88* 3.Fraktion | Vivapur® 200 |

| | | |
|---|---|---|
| *: vermahlenes PVA | | |

### 2b. Abmischungen mit hochdispersem Siliciumdioxid

Zur Verbesserung des Rieselfähigeit werden den Beispielen und Vergleichen jeweils 0,25 Gew.% hochdisperses Siliciumdioxid zugegeben und nochmals 5 Minuten vermischt.

### 2c. Verpressung dieser Abmischungen und Tablettencharakterisierung

Allg. Beschreibung: je 498,75 g der oben hergestellten Co-Mischungen aus den Beispielen A3 und A4 bzw. den Vergleichen B3, C3, B4 und C4 werden in einem Turbulamischgefäß mit 1,25 g Magnesiumstearat versetzt, nochmals 5 min. in einem Turbulamischer T2A gemischt und an einer Exzenterpresse Korsch EK 0-DMS tablettiert.

**Tabelle 2b: Tablettierdaten der Co-Mischungen dergemahlenen PVA-Fraktionen mit der mikrokristallinen Cellulose Vivapur® 200**

| Legende: | | | | | | | |
|---|---|---|---|---|---|---|---|
| A: Presskraft | | [kN] | B: Tablettenhärte nach 1 Tag | | | [N] | |
| C: Tablettenmasse | | [mg] | D: Tablettenhöhe | | | [mm] | |
| E: Abrieb | | [%] | F: Ausstoßkraft | | | [N] | |

| **Muster** | **A** | | **B** | **C** | **D** | **E** | **F** |
|---|---|---|---|---|---|---|---|
| | **Soll** | **Ist** | | | | | |
| **Beispiel A3** | 5 | 5,1 | 72,7 | 494,3 | 5,6 | 0,19 | 108,1 |
| | 10 | 10,0 | 183,5 | 493,5 | 4,9 | 0 | 115,7 |
| | 20 | 19,9 | 387,8 | 495,8 | 4,5 | 0 | 74,4 |
| | 30 | 29,6 | 507,8 | 494,2 | 4,3 | 0 | 59,1 |
| **Vergleich B3** | 5 | 5,0 | 58,0 | 500,4 | 5,6 | 0,27 | 102,2 |
| | 10 | 9,9 | 145,0 | 500,2 | 5,0 | 0 | 109,6 |
| | 20 | 20,2 | 309,1 | 501,4 | 4,5 | 0 | 72,7 |
| | 30 | 29,7 | 416,6 | 502,5 | 4,5 | 0 | 60,6 |
| **Vergleich C3** | 5 | 5,0 | 39,7 | 495,6 | 5,5 | 1,53 | 98,6 |
| | 10 | 10,1 | 100,3 | 495,6 | 4,9 | 0,01 | 109,2 |
| | 20 | 20,9 | 211,4 | 497,8 | 4,5 | 0 | 79,0 |
| | 30 | 30,1 | 295,4 | 497,6 | 4,4 | 0 | 68,2 |
| **Beispiel A4** | 5 | 5,0 | 70,1 | 498,9 | 5,5 | 0,27 | 108,7 |
| | 10 | 9,8 | 179,6 | 499,8 | 4,8 | 0 | 119,3 |
| | 20 | 20,9 | 391,4 | 501,6 | 4,4 | 0 | 75,8 |
| | 30 | 29,5 | 491,6 | 503,1 | 4,3 | 0 | 62,9 |
| **Vergleich B4** | 5 | 5,1 | 53,2 | 497,0 | 5,7 | 0,42 | 105,8 |
| | 10 | 10,0 | 138,7 | 498,0 | 5,0 | 0,01 | 118,6 |
| | 20 | 18,8 | 281,7 | 493,6 | 4,6 | 0 | 82,4 |
| | 30 | 29,1 | 389,4 | 491,1 | 4,5 | 0 | 64,9 |
| **Vergleich C4** | 5 | 5,3 | 35,5 | 498,7 | 5,7 | 1,96 | 92,3 |
| | 10 | 10,2 | 87,8 | 501,1 | 5,1 | 0 | 103,0 |
| | 20 | 19,3 | 176,7 | 502,6 | 4,7 | 0 | 80,7 |
| | 30 | 28,5 | 242,2 | 502,9 | 4,6 | 0 | 67,4 |

In der Figur 2 sind die stark unterschiedlichen Presskraft-Tablettenhärte-Profile zur besseren Veranschaulichung grafisch dargestellt.

## Patentansprüche

1. Vormischung zur Herstellung von wirkstoffhaltigen Tabletten, enthaltend eine Co-Mischung aus Polyvinylalkoholen (PVAs), welche mittlere Korngrößen <100 µm aufweisen, und mikrokristallinen Cellulosen (MCCs).

2. Vormischung gemäß Anspruch 1, enthaltend mikrokristalline Cellulosen mit einer mittleren Korngröße von ≤150 µm, bevorzugt mit einer mittleren Korngröße im Bereich von 100 µm bis 140 µm.

3. Vormischung gemäß der Ansprüche 1 oder 2, enthaltend Polyvinylalkohole mit einer mittleren Korngröße im Bereich von 80 µm bis 90 µm.

4. Vormischung gemäß Anspruch 1, 2 oder 3, **dadurch gekennzeichnet, dass** es sich um eine direkt verpressbare Co-Mischung handelt.

5. Vormischung gemäß einem oder mehreren der Ansprüche 1 bis 4, **dadurch gekennzeichnet, dass** es sich um Co-Mischungen aus PVA mit mikrokristallinen Cellulosen im Gewichtsverhältnis 2:1 bis 1:2, bevorzugt in einem Verhältnis von 2:1 bis 1:1 handelt.

6. Vormischung gemäß einem oder mehreren der Ansprüche 1 bis 5, **dadurch gekennzeichnet, dass** es sich um Co-Mischungen aus mikrokristallinen Cellulosen (MCCs) und zur Wirkstoffretardierung geeigneten Polyvinylalkoholen (PVAs) handelt, wobei letztere die Anforderungen der Pharmakopöen (Ph. Eur., USP/NF und JPE erfüllen.

7. Vormischung gemäß einem oder mehreren der Ansprüche 1, bis 6, **dadurch gekennzeichnet, dass** es sich um Co-Mischungen aus mikrokristallinen Cellulosen (MCCs) und zur Wirkstoffretardierung geeigneten Polyvinylalkoholen (PVAs) der Typen 18-88, 26-88 und 40-88 gemäß den Anforderungen der Pharmakopöen Ph. Eur., USP/NF und JPE, sowie des Typs 28-99 gemäß den Anforderungen der JPE und Ph. Eur. handelt.

8. Vormischung gemäß einem oder mehreren der Ansprüche 1 bis 6, **dadurch gekennzeichnet, dass** es sich um Co-Mischungen aus mikrokristallinen Cellulosen (MCCs) und zur Wirkstoffretardierung geeigneten Polyvinylalkoholen (PVAs) der Typen PVA 26-88 und PVA 40-88 handelt.

9. Vormischung gemäß einem oder mehreren der Ansprüche 1 bis 8, welche durch Kompression bei einer Preßkraft von 20 kN zu Tabletten mit Härten von >380 N geformt werden kann, die und eine Friabilität ≤0.1 Gew.% aufweisen.

10. Vormischung aus feinkörnigem PVA und feinkörnigem MCC gemäß einem oder mehreren der Ansprüche 1 bis 8, welche durch Kompression bei einer Preßkraft von 10 kN zu Tabletten mit Härten von >178 N geformt werden kann, die eine Friabilität ≤0.1 Gew.% aufweisen.

11. Wirkstoffhaltige Tablette, enthaltend eine Trägermatrix bestehend aus einer Vormischung aus feinkörnigem PVA und feinkörnigem MCC gemäß einem oder mehreren der Ansprüche 1 bis 10.

12. Wirkstoffhaltige Tablette gemäß Anspruch 11 mit verlängerter Wirkstofffreisetzung, enthaltend eine Vormischung aus mikrokristallinen Cellulosen (MCCs) und Polyvinylalkoholen (PVAs) gemäß einem oder mehreren der Ansprüche 1 bis 8.

13. Wirkstoffhaltige Tabletten gemäß Anspruch 9, enthaltend eine direkt verpressbare Vormischung gemäß einem oder mehreren der Ansprüche 1 bis 8 in einer Menge von 1 - 99 Gew.-%, bevorzugt in einer Menge von 5 - 95 Gew.-%, ganz besonders bevorzugt in einer Menge von 10 - 90 Gew.-% bezogen auf das Gesamtgewicht der Tablette.

14. Wirkstoffhaltige Tabletten gemäß Anspruch 9 oder 10, welche durch Kompression bei niedrigen Preßkräften mit hohe Tablettenhärten erhalten werden und geringe Ausstoßkräfte erfordern.

15. Wirkstoffhaltige Tabletten gemäß Anspruch 11, welche durch Kompression bei einer Preßkraft von 20 kN mit Härten von >380 N erhalten werden und eine Friabilität ≦0.1 Gew.% aufweisen.

16. Wirkstoffhaltige Tabletten gemäß Anspruch 11, welche durch Kompression bei einer Preßkraft von 10 kN Tabletten mit Härten von >178 N erhalten werden und eine Friabilität ≦0.1 Gew.% aufweisen.

17. Wirkstoffhaltige Tabletten gemäß einem oder mehreren der Ansprüche 11 bis 15 mit verzögerter Wirkstofffreisetzung, enthaltend mit Wirkstoffen der BCS Klasse I entweder allein oder in Kombination mit anderen Wirkstoffen.

## Claims

1. Premix for the production of active compound-containing tablets, comprising a co-mixture of polyvinyl alcohols (PVAs) which have average particle sizes < 100 µm, and microcrystalline celluloses (MCCs).

2. Premix according to Claim 1, comprising microcrystalline celluloses having an average particle size of ≤ 150 µm, preferably having an average particle size in the range from 100 µm to 140 µm.

3. Premix according to Claim 1 or 2, comprising polyvinyl alcohols having an average particle size in the range from 80 µm to 90 µm.

4. Premix according to Claim 1, 2 or 3, **characterised in that** it is a directly compressible co-mixture.

5. Premix according to one or more of Claims 1 to 4, **characterised in that** it is a co-mixture of PVA with microcrystalline celluloses in the weight ratio 2:1 to 1:2, preferably in a ratio of 2:1 to 1:1.

6. Premix according to one or more of Claims 1 to 5, **characterised in that** it is a co-mixture of microcrystalline celluloses (MCCs) and polyvinyl alcohols (PVAs) that are suitable for active compound retardation, where the latter meet the requirements of the pharmacopoeias Ph. Eur., USP/NF and JPE.

7. Premix according to one or more of Claims 1, to 6, characterised it is a co-mixture of microcrystalline celluloses (MCCs) and polyvinyl alcohols (PVAs), suitable for active compound retardation, of grades 18-88, 26-88 and 40-88 in accordance with the requirements of the pharmacopoeias Ph. Eur., USP/NF and JPE, and of grade 28-99 in accordance with the requirements of JPE and Ph. Eur.

8. Premix according to one or more of Claims 1 to 6, **characterised in that** it is a co-mixture of microcrystalline celluloses (MCCs) and polyvinyl alcohols (PVAs) of grades PVA 26-88 and PVA 40-88 that are suitable for active compound retardation.

9. Premix according to one or more of Claims 1 to 8, which can be shaped by compression at a pressing force of 20 kN to give tablets having hardnesses of > 380 N which have a friability of ≤ 0.1% by weight.

10. Premix of fine-grained PVA and a fine-grained MCC according to one or more of Claims 1 to 8 which can be shaped by compression at a pressing force of 10 kN to give tablets having hardnesses of > 178 N which have a friability of ≤ 0.1% by weight.

11. Active compound-containing tablet comprising an excipient matrix consisting of a premix of fine-grained PVA and fine-grained MCC according to one or more of Claims 1 to 10.

12. Active compound-containing tablet according to Claim 11 having extended release of active compound, comprising a premix of microcrystalline celluloses (MCCs) and polyvinyl alcohols (PVAs) according to one or more of Claims 1 to 8.

13. Active compound-containing tablets according to Claim 9, comprising a directly compressible premix according to one or more of Claims 1 to 8 in an amount of 1 - 99% by weight, preferably in an amount of 5 - 95% weight, very particularly preferably in an amount of 10 - 90% by weight, based on the total weight of the tablet.

14. Active compound-containing tablets according to Claim 9 or 10 which are obtained with high tablet hardnesses by compression at low pressing forces and require low ejection forces.

15. Active compound-containing tablets according to Claim 11 which are obtained with hardnesses of > 380 N by compression at a pressing force of 20 kN and have a friability of ≤ 0.1% by weight.

16. Active compound-containing tablets according to Claim 11 which are obtained with hardnesses of > 178 N by compression at a pressing force of 10 kN and have a friability of ≤ 0.1% by weight.

17. Active compound-containing tablets according to one or more of Claims 11 to 15 having delayed release of active compound, comprising active compounds from BCS class I, either alone or in combination with other active compounds.

## Revendications

1. Prémélange destiné à la production de comprimés contenant un composé actif, comprenant un co-mélange d'alcools polyvinyliques (PVA) qui possèdent des tailles de particule moyennes < 100 µm, et de celluloses microcristallines (MCC).

2. Prémélange selon la revendication 1, comprenant des celluloses microcristallines ayant une taille de particule moyenne de ≤ 150 µm, préférablement ayant une taille de particule moyenne dans la plage allant de 100 µm à 140 µm.

3. Prémélange selon la revendication 1 ou 2, comprenant des alcools polyvinyliques ayant une taille de particule moyenne dans la plage allant de 80 µm à 90 µm.

4. Prémélange selon la revendication 1, 2 ou 3, **caractérisé en ce qu'**il s'agit d'un co-mélange directement compressible.

5. Prémélange selon l'une ou plusieurs parmi les revendications 1 à 4, **caractérisé en ce qu'**il s'agit d'un co-mélange de PVA avec des celluloses microcristallines selon un rapport pondéral allant de 2:1 à 1:2, préférablement selon un rapport allant de 2:1 à 1:1.

6. Prémélange selon l'une ou plusieurs parmi les revendications 1 à 5, **caractérisé en ce qu'**il s'agit d'un co-mélange de celluloses microcristallines (MCC) et d'alcools polyvinyliques (PVA) qui sont convenables pour le retardement de composé actif, ce dernier remplissant les exigences des pharmacopées Ph. Eur., USP/NF et JPE.

7. Prémélange selon l'une ou plusieurs parmi les revendications 1 à 6, **caractérisé en ce qu'**il s'agit d'un co-mélange de celluloses microcristallines (MCC) et d'alcools polyvinyliques (PVA), convenables pour le retardement de composé actif, de qualités 18-88, 26-88 et 40-88 conformément aux exigences des pharmacopées Ph. Eur., USP/NF et JPE, et de qualité 28-99 conformément aux exigences de JPE et Ph. Eur.

8. Prémélange selon l'une ou plusieurs parmi les revendications 1 à 6, **caractérisé en ce qu'**il s'agit d'un co-mélange de celluloses microcristallines (MCC) et d'alcools polyvinyliques (PVA) de qualités PVA 26-88 et PVA 40-88 qui sont convenables pour le retardement de composé actif.

9. Prémélange selon l'une ou plusieurs parmi les revendications 1 à 8, qui peut être façonné par compression selon une force de pression de 20 kN afin de conduire à des comprimés ayant des duretés > 380 N et qui possèdent une friabilité de ≤ 0,1% en poids.

10. Prémélange de PVA à grains fins et d'une MCC à grains fins selon l'une ou plusieurs parmi les revendications 1 à 8, qui peut être façonné par compression selon une force de pression de 10 kN afin de conduire à des comprimés ayant des duretés > 178 N et qui possèdent une friabilité de ≤ 0,1% en poids.

11. Comprimé contenant un composé actif, comprenant une matrice d'excipient constituée d'un prémélange de PVA à grains fins et de MCC à grains fins selon l'une ou plusieurs parmi les revendications 1 à 10.

12. Comprimé contenant un composé actif selon la revendication 11, présentant une libération prolongée de composé actif, comprenant un prémélange de celluloses microcristallines (MCC) et d'alcools polyvinyliques (PVA) selon l'une ou plusieurs parmi les revendications 1 à 8.

13. Comprimés contenant un composé actif selon la revendication 9, comprenant un prémélange directement compressible selon l'une ou plusieurs parmi les revendications 1 à 8 selon une quantité de 1 - 99% en poids, préférablement selon une quantité de 5 - 95% en poids, très particulièrement préférablement selon une quantité de 10 - 90% en poids, sur la base du poids total du comprimé.

14. Comprimés contenant un composé actif selon la revendication 9 ou 10, qui sont obtenus avec des duretés de comprimé élevées par compression à des forces de pression faibles, et nécessitent de faibles forces d'éjection.

15. Comprimés contenant un composé actif selon la revendication 11, qui sont obtenus avec des duretés de > 380 N par compression à une force de pression de 20 kN, et possèdent une friabilité de ≤ 0,1% en poids.

16. Comprimés contenant un composé actif selon la revendication 11, qui sont obtenus avec des duretés de > 178 N par compression à une force de pression de 10 kN, et possèdent une friabilité de ≤ 0,1% en poids.

17. Comprimés contenant un composé actif selon l'une ou plusieurs parmi les revendications 11 à 15, présentant une libération retardée de composé actif, comprenant des composés actifs de classe I du BCS, seuls ou bien en combinaison avec d'autres composés actifs.
